# EUROPEAN PATENT SPECIFICATION

(11) **EP 3 441 064 B1**
(45) Date of publication and mention of the grant of the patent: **28.07.2021**
(21) Application number: 18188605.2
(22) Date of filing: 11.08.2018
(51) Int. Cl.: A61K 9/08, A61K 9/10, A61K 9/50, A61K 31/09, A61P 11/10

(54) **EXTENDED RELEASE LIQUID COMPOSITIONS OF GUAIFENESIN**
FLÜSSIGKEITSZUSAMMENSETZUNGEN AUS GUAIFENESIN MIT VERLÄNGERTER FREISETZUNG
COMPOSITIONS LIQUIDES À LIBÉRATION PROLONGÉE DE GUAIFÉNÉSINE

(30) Priority: 11.08.2017 IN 201711028571
(43) Date of publication of application: 13.02.2019
(73) Proprietor: Sun Pharmaceutical Industries Limited, 400063 Mumbai (IN)
(72) Inventor: VATS, Sandeep Kumar, 131001 Haryana (IN); Vankayala, Radhakrishna, 522601 Andhra Pradesh (IN); Mondal, Balaran, 721134 West Bengal (IN); SINGH, Romi Barat, 221002 Varanasi, Uttar Pradesh (IN); KUMAR, Ashish, 124507 Jhajjar (IN); Ramaraju, Kalaiselvan, Trichirapalli, Tamil Nadu 521999 (IN)
(74) Representative: HGF

(56) References cited:
- WO-A1-2018/055641
- WO-A2-2011/107855
- US-A1- 2013 149 383
- US-A1- 2016 271 070

## Description

### Field of the Invention

The present invention relates to an extended release liquid compositions of guaifenesin present in amount of 75 mg/mL to 200 mg/mL wherein the composition is to be given once or twice daily only and exhibits reduced food effect when administered in a healthy human subjects in a fasting or a fed state.

### Background of the Invention

Guaifenesin is an expectorant drug widely used to relieve chest and nasal congestion. The expectorant effects of guaifenesin promote elimination of mucous by thinning the mucous and lubricating the irritated respiratory tract.

Guaifenesin has a short plasma half-life of about 1 hour. The immediate release preparations such as tablets as well as syrups have to be administered multiple times daily and provide relief only for a shorter duration of time. This leads to patient incompliance and also causes great inconvenience to the patients and caregivers especially in cases of pediatric population.

Further, another problem associated with the guaifenesin immediate release formulation is the effect of food which is explicitly observed in the liquid formulation of guaifenesin which affects the bioavailability of the drug when taken with meal.

Extended release preparations of guaifenesin which provide prolonged therapeutic effectare highly desirable.

U.S. Patent No. 7,838,032 discloses anextended release tablet of guaifenesin having two portions, wherein a first portion comprises guaifenesin in an immediate release form, and a second portion comprises guaifenesin in an extended release form, and wherein the tablet contains 1200 mg of guaifenesin.

U.S. Publication No. 2013/0149383 discloses a composition comprising plurality of particles, each particle comprising guaifenesin, a hydrophobic wax matrix, a stabilizer and a release modifier; wherein the plurality of particles have a mean particle size diameter of from about 20 µm to about 500 µm.

Extended release tablets of guaifenesin are marketed under the brand name Mucinex® in strengths of 600 mg and 1200mg. Mucinex® is a non-scored, bi-layer tablet and provides relief for twelve hours.Being anextended release non-scored tablet, it is difficult to divide tablets in order to provide flexible dosage regimen especially in case of pediatrics. Further, prescription information of Mucinex® also prohibits crushing, breaking or chewing the extended release tablet since doing this may ultimately lead to the loss of extended release behavior leading to dose dumping. Owing to this, the large sized tablet of around 22 mm size has to be administered fully, which makes it difficult for the patients to swallow, particularly patients suffering from dysphagia, pediatric patients and geriatric patients.

Prior art documents which describe extended release liquid formulations are WO 2018/055641 A1, WO 2011/107855 A2, US 2016/271070 A1 and US 2013/149383 A1. There is an unmet need of an easy to swallow extended release composition of guaifenesin which ensures patient compliance by reducing dose frequency and which is easy to administer to dysphagia, pediatric and geriatric patients.

In view of all this, extended release liquid compositions provide the best alternative over the extended release solid compositions.

However, since guaifenesin is a BCS Class I drug and is freely soluble in aqueous media and many other solvents including acetone, formulating a liquid composition of guaifenesin with extended release coating is challenging, as the drug tends to get leached out from the extended release portion into the vehicle during storage.

Further, a larger amount of release controlling excipients would be needed to control release and prepare extended release formulation. A high ratio of release controlling excipients to guaifenesin would pose challenge of high solid content per ml of dosage form leading to dosing and palatability challenges. Alternatively, if the solid content is reduced by adding more vehicle for ease of measurement and dosing, the total volume to be administered for single dose would increase. This would again pose a challenge of patient compliance, especially for pediatric population. It would also lead to a bulky pack to contain the complete dosing for a treatment duration.

Hence, the present inventors have developed extended release composition of guaifenesin having improved and desired characteristics that exhibits a reduced food effect.

### Summary of the Invention

The present invention relates to an extended release liquid compositions of guaifenesin which exhibits a reduced food effect when administered in a healthy human subjects in a fasting or a fed state.

The extended release liquid compositions are in the form of suspensions which are ready-to-use or suspensions which are reconstituted from powder. The compositions represent a significant advancement as they are stable, easy to administer, provide extended release, and are readily dose titrable. The extended release suspension compositions provide consistency of the release profile during the shelf life and mitigate the risk of premature release or dose dumping of guaifenesin.

### Detailed Description of the Invention

The present invention provides extended release liquid compositions comprising guaifenesin as the active agent for once or twice daily administration, wherein the composition release the active agent guaifenesin *in-vivo* for a prolonged duration without compromising the bioavailability of the guaifenesin and also provides a better patient compliance by reducing the frequency of the dosage form. Further, the dosage form compositions of the present invention is designed in such a manner that dissolution behavior does not depend on the state of digestion. Still further, the compositions are also capable of providing an initial rapid effect followed by a prolonged effect of the active agent for use in certain situations where such combined effect is desirable.

A first aspect of the present invention provides an extended release liquid composition comprising guaifenesin in amount of about 75 mg/mL to about 200 mg/mL, wherein the composition exhibits reduced food effect when administered in a healthy human subjects in a fasting or a fed state.

According to another embodiment of this aspect, the extended release liquid composition, when administered to healthy subjects, provides a mean *C*ₘₐₓ value in the range of about 1.0 to about 2.0 ng/mL/mg.

According to another embodiment of this aspect, the extended release liquid composition provides a mean AUC₀₋ₜvalue in the range of about 4.0 to about 8.0 ng.hr/mL/mg.

According to another embodiment of the above aspect, the extended release liquid composition comprises guaifenesin in an extended release portion and an immediate release portion.

According to another embodiment of the above aspect, the ratio of guaifenesin in an extended release portion to immediate release portion is from about 50:50 to about 100:0.

According to another embodiment of the above aspect, the ratio of guaifenesin in an extended release portion to immediate release portion is from about 75: 25 to about 85:15.

According to another embodiment of the above aspect, the extended release portion comprises a core comprising guaifenesin having an extended release coating of one or more release-controlling excipients.

A second aspect of the present invention provides an extended release liquid composition comprising a core comprising guaifenesin and an extended release coating, wherein the thickness of the extended release coating per unit surface area is from 3× 10⁻³ to 7× 10⁻³ micron/mm^{2.}

According to the invention, the extended release liquid composition of guaifenesin comprises:
(i) an extended release portion comprising cores of guaifenesin having coating of one or more release controlling excipients,
(ii) an immediate release portion, and
(iii) a pharmaceutically acceptable vehicle,
wherein guaifenesin is present in an amount of 75 mg/ml to 200 mg/ml,
wherein guaifenesin is present in the cores or is layered over inert particles to form drug coated cores,
wherein the cores or the drug coated cores further comprise: an osmogent selected from xylitol, mannitol, glucose, lactose, sucrose or sodium chloride; and a stabilizer selected from alginic acid, sodium alginate, guar gum, locust bean gum, tragacanth, carrageenan, gum acacia, gum arabic, gellan gum, sterculia gum, karaya gum or xanthan gum.

According to another embodiment, the extended release liquid composition comprises:
(i) an extended release portion comprising cores of guaifenesin having a coatingof one or more release-controlling excipients;
(ii) an immediate release portion coated over the extended release portion
(iii) a vehicle.

According to another embodiment of this aspect, the guaifenesin may be present in the core or layered over an inert particle.

According to another embodiment of this aspect, the inert particle is selected from a group consisting of a non-pareil seed, a microcrystalline cellulose sphere, a dibasic calcium phosphate bead, a mannitol bead, a silica bead, a tartaric acid pellet, and a wax based pellet.

According to another embodiment of this aspect, the d₉₀ of coated cores comprising guaifenesin is from about 1 µm to about 700 µm, particularly from about 50 µm to about 500 µm, and more particularly from about 100 µm to about 400µm.

According to another embodiment of this aspect, the d₉₀ of coated cores comprising guaifenesin and coated with a release-controlling excipient ranges from about 10 µm to about 1000 µm, particularlyfrom about 100 µm to about 700 µm, and more particularly from about 200 µm to about 500 µm.

According to another embodiment of this above aspect, the core of the extended release portion comprising guaifenesin may further comprise osmoagents and stabilizers.

According to another embodiment of the above aspect, osmoagentis water soluble osmoagents and may be present in amount of about0.1 mg/ml to 50 mg/ml.

According to another embodiment of this aspect, the release-controlling excipient is selected from the group consisting of a pH-dependent release controlling agent, a pH-independent release controlling agent, and mixtures thereof.

According to another embodiment of the above aspect, the *in-vitro* dissolution release profile of the extended release liquid composition remains substantially similar to the initial *in-vitro* dissolution release profile upon storage for at least seven days.

According to another embodiment of this aspect, the composition is characterized by having an osmolality ratio of at least about 1.

According to another embodiment of the above aspect, the extended release liquid composition is characterized by having an *in-vitro* dissolution release profile as determined by USP type 2 apparatus at 100 rpm, in 900 mL of 0.1N HCL at 37°C as follows:
- not less than 10% of guaifenesin is released at 1 hour,
- about 40-80% of guaifenesin is released at 4 hours, and
- not less than 80% of guaifenesin is released at 12 hours.

According to another embodiment of the above aspect, the extended release liquid composition is characterized by having an *in-vitro* dissolution release profile as determined by USP type 2 apparatus at 100 rpm, in 900 mL of phosphate buffer (pH 6.8) at 37°C as follows:
- not less than 10% of guaifenesin is released at 1 hour,
- about 40-80% of guaifenesin is released at 4 hours, and
- not less than 80% of guaifenesin is released at 12 hours.

According to another embodiment of the above aspect, the dose of guaifenesin ranges from about 600 mg to about 1200 mg twice daily.

The extended release liquid composition of guaifenesin comprises guaifenesin in a concentration from about 75 mg/mL to about 200 mg/mL of the composition. More particularly, the extended release liquid composition of guaifenesin comprises guaifenesin in a concentration from about 100 mg/mL to about 200 mg/mL of the composition.Particularly, the extended release liquid composition of guaifenesin comprises guaifenesin in a concentration of about 72 mg/mL, about 90 mg/mL, about 100 mg/mL, and about 108 mg/mL.

According to another embodiment of the above aspect, the extended release liquid compositions are in the form of suspensions which are ready-to-use or suspensions which are reconstituted from powder.

According to another embodiment of the above aspect, the extended release composition is packaged in a bottle or a sachet.

According to another embodiment of this aspect, the extended release composition is packaged in a dual chamber pack.

According to another embodiment of the above aspect, the suspension powder for reconstitution is prepared by a process comprising the steps of:
(i) preparing cores comprising inert particles coated with guaifenesin, and optionally comprising osmoagents, stabilizers and one or more pharmaceutically acceptable excipients;
(ii) dissolving/dispersing release controlling excipients and one or more pharmaceutically acceptable coating additives in a suitable solvent;
(iii) applying the coating composition of step (ii) over the cores of step (i) to form an anextended release portion; and
(iv)packing the coated cores of step (iv) in a suitable pack.
Before administration, the suspension powder for reconstitution is reconstituted with a vehicle comprising guaifenesin, one or more osmogents and pharmaceutically acceptable excipients to obtain the extended release liquid composition.

According to another embodiment of the above aspect, the suspension powder for reconstitution is prepared by a process comprising the steps of:
(i) preparing cores comprising guaifenesin, and optionally osmoagents, stabilizers and one or more pharmaceutically acceptable excipients;
(ii) dissolving/dispersing a release controlling excipients and one or more pharmaceutically acceptable coating additives in a suitable solvent;
(iii) applying the coating composition of step (ii) over the cores of step (i) to form an extended release portion;
(iv) dissolving/dispersing guaifenesin and stabilizer in a suitable solvent;
(v) applying the coating composition of step (iv) over the cores of step (iii); and
(vi) packing the coated cores of step (v) in a suitable pack.
Before administration, the suspension powder for reconstitution is reconstituted with a vehicle comprising one or more osmogents and pharmaceutically acceptable excipients to obtain the extended release liquid composition.

According to another embodiment of the above aspect, the extended release liquid composition is bioequivalent to Mucinex® tablets containing 1200 mg of guaifenesin.

A third aspect of the present invention provides a low dose extended release liquid composition comprising guaifenesin.

The term "guaifenesin," as used herein, refers to guaifenesin, as well as its pharmaceutically acceptable salts, polymorphs, hydrates, solvates, prodrugs, chelates, esters, and complexes. The guaifenesin is present in the extended release liquid composition in an amount from about 2% to about 40% w/v based on total weight of the composition, particularly in an amount of about 5% w/v to about 20% w/v based on total weight of the composition.

The term "extended release," as used herein, refers to a release profile of guaifenesin over an extended period of time, *e.g.,* over a period that includes for example, 4, 6, 8, 12, 24 hours, or more. The extended release includes sustained release, controlled release, multiphase release, delayed release, pulsatile release, chrono release and the like.

The term "immediate release," as used herein, refers to a dose of guaifenesin that is substantially completely released with a time period of about 1 hour or less for example, more particularly about 45 minutes or less.

The immediate release portion helps in providing an immediate therapeutic effect which is subsequently followed by an extended therapeutic effect. The guaifenesin is present in the immediate release form in an amount from about 0% to about 30% w/v based on the total weight of the composition, particularly in an amount of about 1% w/v to about 20% w/v based on total weight of the composition, and more particularly in an amount of about 1.0%w/v to about 10 % w/v based on total weight of the composition.

The ratio of the extended release portion to immediate release portion of guaifenesin is significant in preparing the extended release liquid composition of guaifenesin because of very high solubility of guaifenesin. The amount of extended release portion and immediate release portion is adapted so as to maintain a balance such that a predetermined dissolution characteristic of the novel extended release composition is obtained as well as there will be no rupturing problem. The ratio of the extended release portion to immediate release portion of guaifenesin in the extended release liquid compositions is from about 50:50 to about 100:0. Particularly, the ratio of the extended release portion to immediate release portion of guaifenesin in the extended release liquid compositions is from about 60:40 to about 95:5. More particularly, the ratio of the extended release portion to immediate release portion of guaifenesinin the extended release liquid compositionsis from about 70:30 to about 95:10.Still more particularly, the ratio of the extended release portion to immediate release portion of guaifenesin is from about75: 25 and about 85:15.

Pharmacokinetic parameters such as Cₘₐₓ, Tₘₐₓ, AUC_{0→t}and AUC_{0→∞} are used to establish bioequivalency.

The *in-vivo* release profile can be assessed using pharmacokinetic parameters of Cₘₐₓ, Tₘₐₓ and area under the curve (AUC).

The term "Cₘₐₓ" as used herein refers to the maximum plasma concentration of guaifenesin.

The term "Tₘₐₓ" as used herein, refers to the time to reach maximum plasma concentration of guaifenesin.

The term "AUC_{0-∞}" as used herein, refers to the area under the plasma concentration-time curve extrapolated to infinity.

The term "AUC₀₋ₜ" as used herein, refers to the area under the plasma concentration-time curve till time t.

The pharmacokinetic parameters are calculated as mean values taken from a population of individuals participating in the study.

The extended release liquid compositions also provide the consistent *in-vivo* release which ensures steady and predictable guaifenesin release with minimal inter and intra subject variation throughout the shelf life of the composition.

The term "reduced food effect," as used herein, refers to a small relative difference in AUC and Cₘₐₓ of a drug, when said drug or a formulation thereof is administered orally to a human concomitantly with food or in a fed state as compared to when the same formulation is administered in a fasted state or without food. Reduced food effect herein means AUC₀₋ₜbetween 0.8 to 1.2 or both under fed and fasted state.

The suspension powder for reconstitution having coated cores of guaifenesin may be reconstituted with the vehicle comprising suspending agents, osmogents, pharmaceutically acceptable excipients, and a pharmaceutically acceptable solvents. Alternatively, suspending agents, osmogents, or other pharmaceutically acceptable excipients may be premixed with the coated cores which may be reconstituted with an aqueous vehicle. In case of powder for suspension, the vehicle may be pre-formed or formed at the time of reconstitution.

The pharmaceutically acceptable vehicle can include a saturated solution of guaifenesin for immediate release.

The term "about," as used herein, refers to any value which lies within the range defined by a variation of up to ±10% of the value.

The term "equivalent" as used herein, refers to any value which lies within the range defined by a variation of up to ±30% of the value.

The term "significant leaching," as used herein means more than 20% of the guaifenesin is leached out from the coated cores into the solution.

The term "substantial," as used herein refers to any value which lies within the range as defined by a variation of up to ±15 from the average value.

The term "stable," as used herein, refers to chemical stability, wherein not more than 5% w/w of total related substances are formed on storage at 40°C and 75% relative humidity (R.H.) or at 25°C and 60% R.H. for a period of at least three months, particularly for a period of six months. Further, stable as used herein, refers therein no significant change in assay and dissolution when stored at 40°C/75% R.H.

The cores comprise one or more pharmaceutically acceptable excipients such as a stabilizer,osmoagents,and a binder.

Guaifenesin may be present in the core or layered over an inert particle to form a core. The inert particle is insoluble in water. When there is drug layering over the core, the stabilizers and osmoagents may be incorporated into the drug layer. The addition of stabilizers in the core coated with a release controlling excipient help in stabilization of the coating by suppression of undesired guaifenesin movement from the core to the extended release coating thereby maintaining the integrity of the coated cores. Suitable stabilizers are selected from the group comprising gums and their derivatives such as alginic acid, sodium alginate, guar gum, locust bean gum, tragacanth, carrageenan, gum acacia, gum arabic, gellan gum,sterculia gum,karaya gum, and xanthan gum. Other stabilizers can be cellulose derivatives such as ethyl cellulose, hydroxypropyl cellulose, hydroxypropylmethyl cellulose, methylcellulose, sodium carboxymethyl cellulose, hydroxyethyl cellulose; polyvinylpyrrolidone; polyvinyl alcohol; polyacrylic acid; polymethacryic acid; carboxyvinyl polymers; gelatin; pregelatinized starch; agar; polyethylene glycols; and mixtures thereof. The stabilizer is present in an amount of 0.5% to 40% w/w based on the total weight of the drug layered cores.

The term "osmogent," as used herein, refers to all pharmaceutically acceptable inert water-soluble compounds that can imbibe or dissolve in water and/or aqueous biological fluids. Suitable examples of osmogents or pharmaceutically acceptable inert water-soluble compounds arc selected from the group comprising carbohydrates such as xylitol, mannitol, sorbitol, arabinose, ribose, xylose, glucose, fructose, mannose, galactose, sucrose, maltose, lactose, dextrose, and raffinose; water-soluble salts of inorganic acids such as magnesium chloride, magnesium sulfate, potassium sulfate, lithium chloride, sodium chloride, potassium chloride, lithium hydrogen phosphate, sodium hydrogen phosphate, potassium hydrogen phosphate, lithium dihydrogen phosphate, sodium dihydrogen phosphate, potassium dihydrogen phosphate, and sodium phosphate tribasic; water-soluble salts of organic acids such as sodium acetate, potassium acetate, magnesium succinate, sodium benzoate, sodium citrate, and sodium ascorbate; water-soluble amino acids such as glycine, leucine, alanine, and methionine; urea or its derivatives; propylene glycol; glycerin; polyethylene oxide; hydroxypropylmethyl cellulose; and mixtures thereof. The osmogents used in the present invention are xylitol, mannitol, glucose, lactose, sucrose, and sodium chloride.

The osmogents used in the present invention are xylitol, mannitol, glucose, lactose, sucrose, and sodium chloride and are present in amount of about 0.1 mg/ml to 50 mg/ml in the drug coated core of the composition.

The release-controlling excipients used to form the extended release coating are selected from a group comprising a pH-dependent agent, a pH-independent agent, and mixtures thereof. The release-controlling excipient is present in an amount of about 20% to about 80% w/w based on the total weight of the coated cores, particularly from about 20% to about 70% w/w based on the total weight of the coated cores, and more particularly from about 20% to about 60% w/w based on the total weight of the coated cores.

Suitable examples of pH-dependent agents are selected from the group comprising acrylic copolymers for example methacrylic acid and methyl methacrylate copolymers, e.g., Eudragit® L 100 and Eudragit® S 100, methacrylic acid and ethyl acrylate copolymers, e.g., Eudragit® L 100-55 and Eudragit® L 30 D-55, dimethylaminoethyl methacrylate and butyl methacrylate and methyl methacrylate copolymers, *e.g*., Eudragit® E 100 and Eudragit® E PO, methyl acrylate and methacrylic acid and octyl acrylate copolymers, styrene and acrylic acid copolymers, butyl acrylate and styrene and acrylic acid copolymers, and ethylacrylate-methacrylic acid copolymer; cellulose acetate phthalate; cellulose acetate succinates; hydroxyalkyl cellulose phthalates such as hydroxypropylmethyl cellulose phthalate; hydroxyalkyl cellulose acetate succinates such as hydroxypropylmethyl cellulose acetate succinate; vinyl acetate phthalates; vinyl acetate succinate; cellulose acetate trimelliate; polyvinyl derivatives such as polyvinyl acetate phthalate, polyvinyl alcohol phthalate, polyvinyl butylate phthalate, and polyvinyl acetoacetal phthalate; zein; shellac; and mixtures thereof.

Suitable examples of pH-independent agents are selected from the group comprising cellulosic polymers for example ethyl cellulose, methyl cellulose, hydroxyethyl cellulose, hydroxypropyl cellulose, hydroxyethylmethyl cellulose, hydroxypropylmethyl cellulose, and carboxy methylcellulose; acrylic copolymers such as methacrylic acid copolymers, *e.g*., Eudragit® RS, Eudragit® RL, and Eudragit® NE 30 D; cellulose acetate; polyethylene derivatives, e.g., polyethylene glycol and polyethylene oxide; polyvinyl alcohol; polyvinyl acetate; gums, e.g., guar gum, locust bean gum, tragacanth, carrageenan, alginic acid, gum acacia, gum arabic, gellan gum, and xanthan gum; triglycerides; waxes, *e.g*., glyceryl behenate, hydrogenated vegetable oils; lipids; fatty acids or their salts/derivatives; a mixture of polyvinyl acetate and polyvinyl pyrrolidone, *e.g.,* Kollidon® SR; and mixtures thereof. In particular, the pH-independent polymer used in the present invention is ethyl cellulose.

Particularly, ion-exchange resins are not used to obtain the extended release composition of guaifenesin.

Immediate release guaifenesin may be present in the form of a powder, a pellet, a bead, a spheroid, or a granulein the vehicle, or in the form of immediate release coating over the extended release coated cores.

The vehicle further comprises various pharmaceutically acceptable excipients.

The term "pharmaceutically acceptable excipients," as used herein, refers to excipients that are routinely used in pharmaceutical compositions. The pharmaceutically acceptable excipients may comprise glidants, sweeteners, suspending agents, osmogents, anti-caking agents, wetting agents, preservatives, buffering agents, flavoring agents, anti-oxidants, chelating agents, and combinations thereof.

Suitable glidants are selected from the group comprising silica, calcium silicate, magnesium silicate, colloidal silicon dioxide, cornstarch, talc, stearic acid, magnesium stearate, calcium stearate, sodium stearyl fumarate, hydrogenated vegetable oil, and mixtures thereof.

Suitable sweeteners are selected from the group comprising saccharine or its salts such as sodium, potassium, or calcium, cyclamate or its salt, aspartame, alitame, acesulfame or its salt, stevioside, glycyrrhizin or its derivatives, sucralose, and mixtures thereof.

Suitable suspending agents are selected from the group comprising cellulose derivatives such as co-processed spray dried forms of microcrystalline cellulose and carboxymethyl cellulose sodium, hydroxypropyl cellulose, hydroxyethyl cellulose, hydroxypropylmethyl cellulose, methylcellulose, carboxymethyl cellulose and its salts/derivatives, and microcrystalline cellulose; carbomers; gums such as locust bean gum, xanthan gum, tragacanth gum, arabinogalactan gum, agar gum, gellan gum, guar gum, apricot gum, karaya gum, sterculia gum, acacia gum, gum arabic, and carrageenan; pectin; dextran; gelatin; polyethylene glycols; polyvinyl compounds such as polyvinyl acetate, polyvinyl alcohol, and polyvinyl pyrrolidone; sugar alcohols such as xylitol and mannitol; colloidal silica; and mixtures thereof. The co-processed spray dried forms of microcrystalline cellulose and carboxymethyl cellulose sodium have been marketed under the trade names Avicel® RC-501, Avicel® RC-581, Avicel® RC-591, and Avicel® CL-611. The suspending agent is present in an amount of not more than about 20% w/w, based on the total weight of the vehicle.

Suitable anti-caking agents are selected from the group comprising colloidal silicon dioxide, tribasic calcium phosphate, powdered cellulose, magnesium trisilicate, starch, and mixtures thereof.

Suitable wetting agents are selected from the group comprising anionic, cationic, nonionic, or zwitterionic surfactants, and combinations thereof. Suitable examples of wetting agents are sodium lauryl sulphate; cetrimide; polyethylene glycols; polyoxyethylene-polyoxypropylene block copolymers such as poloxamers; polyglycerin fatty acid esters such as decaglyceryl monolaurate and decaglycerylmonomyristate; sorbitan fatty acid esters such as sorbitan monostearate; polyoxyethylene sorbitan fatty acid esters such as polyoxyethylene sorbitan monooleate; polyethylene glycol fatty acid esters such as polyoxyethylene monostearate; polyoxyethylene alkyl ethers such as polyoxyethylene lauryl ether; polyoxyethylene castor oil; and mixtures thereof.

Suitable preservatives are selected from the group comprising parabens such as methyl paraben and propyl paraben; sodium benzoate; and mixtures thereof.

Suitable buffering agents are selected from the group comprising citric acid, sodium citrate, sodium phosphate, potassium citrate, acetate buffer, or mixtures thereof.

Suitable flavoring agents are selected from the group comprising peppermint, grapefruit, orange, lime, lemon, mandarin, pineapple, strawberry, raspberry, mango, passion fruit, kiwi, apple, pear, peach, apricot, cherry, grape, banana, cranberry, blueberry, black currant, red currant, gooseberry, lingon berries, cumin, thyme, basil, camille, valerian, fennel, parsley, chamomile, tarragon, lavender, dill, bargamot, salvia, aloe vera balsam, spearmint, eucalyptus, special compound 501437T and combinations thereof.

Suitable antioxidants are selected from the group comprising butylated hydroxytoluene (BHT), butylated hydroxyanisole (BHA), sodium metabisulfite, ascorbic acid, propyl gallate, thiourea, tocopherols, beta-carotene, and mixtures thereof.

Suitable chelating agents are selected from the group comprising ethylenediamine tetraacetic acid or derivatives/salts thereof, e.g., disodium edetate; dihydroxyethyl glycine; glucamine; acids, e.g., citric acid, tartaric acid, gluconic acid, and phosphoric acid; and mixtures thereof.

Suitable binders are selected from the group comprising polyvinyl pyrrolidone, starch, pregelatinized starch, hydroxypropylmethyl cellulose, hydroxyethyl cellulose, methyl cellulose, sodium carboxymethyl cellulose, gums, acrylate polymers, and mixtures thereof.

The cores of the present invention comprising guaifenesin can be prepared by any method known in the art, e.g., extrusion-spheronoization, wet granulation, dry granulation, hot-melt extrusion granulation, spray drying, and spray congealing. Alternatively, guaifenesin can be layered onto an inert particle to form the core.

Further, guaifenesin can be directly coated with a release-controlling excipients to form the microparticles or microcapsules. The microparticles or microcapsules can be prepared by a process of homogenization, solvent evaporation, coacervation phase separation, spray drying, spray congealing, polymer precipitation, or supercritical fluid extraction.

The extended release liquid compositions of the present invention may further comprise one or more seal coating layers which may be applied before and/or after the extended release coating. The seal coating layer may comprise of one or more film-forming polymers and coating additives.

Examples of film-forming polymers include ethylcellulose, hydroxypropyl methylcellulose, hydroxypropylcellulose, methylcellulose, carboxymethyl cellulose, hydroxymethylcellulose, hydroxyethylcellulose, cellulose acetate, hydroxypropyl methylcellulose phthalate, cellulose acetate phthalate, cellulose acetate trimellitate; waxes such as polyethylene glycol; and methacrylic acid polymers such as Eudragit®. Alternatively, commercially available coating compositions comprising film-forming polymers marketed under various trade names, such as Opadry®, may also be used.

The coating additives used in the present invention are selected from the group comprising plasticizers, opacifiers, anti-tacking agents, coloring agents, and combinations thereof.

Suitable plasticizers are selected from the group comprising triethyl citrate, dibutyl sebacate, triacetin, acetylated triacetin, tributyl citrate, glyceryl tributyrate, diacetylated monoglyceride, rapeseed oil, olive oil, sesame oil, acetyl tributyl citrate, acetyl triethyl citrate, glycerin, sorbitol, diethyl oxalate, diethyl phthalate, diethyl malate, diethyl fumarate, dibutyl succinate, diethyl malonate, dioctyl phthalate, and combinations thereof.

Suitable opacifiers are selected from the group comprising titanium dioxide, manganese dioxide, iron oxide, silicon dioxide, and combinations thereof.

Suitable anti-tacking agents are selected from the group comprising talc, magnesium stearate, calcium stearate, stearic acid, silica, glyceryl monostearate, and combinations thereof.

Suitable coloring agents are selected from the group consisting of FD&C (Federal Food, Drug and Cosmetic Act) approved coloring agents; natural coloring agents; natural juice concentrates; pigments such as iron oxide, titanium dioxide, and zinc oxide; and combinations thereof.

Coating may be performed by applying the coating composition as a solution/suspension/blend using any conventional coating technique known in the art, such as spray coating in a conventional coating pan, fluidized bed processor, dip coating, or compression coating. The percentage of the coating build-up shall be varied depending on the required extended release.

Suitable solvents used for granulation or for forming a solution or dispersion for coating are selected from the group comprising water, ethanol, methylene chloride, isopropyl alcohol, acetone, methanol, and combinations thereof.

The suspension powder for reconstitution may be packaged in a suitable package such as a bottle or a sachet. Further, the sachet can be filled as a unit dose or a multidose sachet. In particular, the sachet pack is a stick pack. The present invention further includes a co-package or a kit comprising two components, wherein one package or one component comprises a suspension powder for reconstitution and another package or another component comprises a vehicle. Alternatively, a dual chamber pack (dual chamber bottle) with two chambers can be used. In this case, one chamber comprises a powder for suspension and another chamber comprises a vehicle. Before administration, the suspension powder for reconstitution and vehicle are mixed to form the extended release suspension compositions.

The extended release liquid compositions have a pH ranging from 4 to 10.

The extended release liquid compositions of guaifenesin have a viscosity ranging from about 500 to 10000 mPas.Particularly, the viscosity of the extended release suspension compositions ranges from about 1000 to 5000 mPas. More particularly, the viscosity of the extended release suspension compositions ranges from about 1500 to 3000 mPas.Much more particularly, the viscosity of the extended release suspension compositions ranges from about 1500 to 1900 mPas. The viscosity can be measured by using a Brookfield Viscometer having a # 2 spindle rotating at 5 rpm at 25°C.

The invention may be further illustrated by the following examples, which are for illustrative purposes only and should not be construed as limiting the scope of the invention in any way.

### EXAMPLES

### Example 1: Extended Release Suspension Powder of Guaifenesin for Reconstitution

| **Ingredients** | **Quantity (mg/mL)** |
|---|---|
| **Drug layered cores** | |
| Guaifenesin | 70.00 |
| Microcrystalline cellulose | 56.00 |
| Sodium alginate | 7.00 |
| Purified water* | q.s. |
| Weight of drug layeredcores | 133.00 |

| **Extended release coating** | |
|---|---|
| Drug layered cores | 133.00 |
| Ethyl cellulose | 39.10 |
| Dibutyl sebacate | 0.80 |
| Acetone* | q.s. |
| Purified water* | q.s. |
| Weight of extended release coated cores | 172.90 |

| **Lubrication** | |
|---|---|
| Extended release coated cores | 172.90 |
| Magnesium stearate | 0.86 |
| Weight of lubricated extended release coated cores | 173.76 |

| **Vehicle** | |
|---|---|
| Guaifenesin(Immediate release portion) | 30.00 |
| Microcrystalline cellulose/carboxymethyl cellulose sodium (Avicel® CL-611) | 20.00 |
| Xanthan gum | 1.50 |
| Colloidal silicon dioxide | 3.50 |
| Sucralose | 0.50 |
| Xylitol | 450.00 |
| Strawberry flavor | 1.00 |
| Purified water | q.s.to 1 mL |

| | |
|---|---|
| *Evaporates during processing | |

### Procedure:

1. Sodium alginate was dissolved in water to a clear solution
2. Guaifenesin was dissolved in the solution of step 1.
3. Microcrystalline cellulose spheres were coated with the solution of step 2 to obtain drug layered cores.
4. Ethyl cellulose and dibutyl sebacate were dispersed in a mixture of acetone and purified water.
5. The drug layered cores of step 3 were coated with the coating dispersion of step 4 to obtain the extended release coated cores.
6. Magnesium stearate was mixed with the extended release coated cores of step 5 to form the suspension powder for reconstitution.
7. Xylitol, guaifenesin (immediate release portion), xanthan gum, microcrystalline cellulose and carboxymethyl cellulose sodium (Avicel® CL -611), colloidal silicon dioxide, and sucralose were dispersed/dissolved in purified water to form a vehicle.
8. The suspension powder for reconstitution of step 6 and vehicle of step 7 were filled separately in a dual chamber bottle.
   Before administration, the contents of the dual chamber bottle can be mixed to form the extended release liquid composition of guaifenesin.

### In-Vitro Studies

The extended release liquid composition prepared as per Example 1 was stored at room temperature for 30 days. The *in-vitro* dissolution was determined at 0, 15, and 30 days using USP type II apparatus at 50 rpm, in 900 mL of 0.1N HCL at 37°C. The results of the release studies are represented in Table 1.

**Table 1: Percentage (%) of the In-Vitro Guaifenesin Release in USP Type II Apparatus (Media: 0.1N HCL, 900 mL, and 50 rpm)**

| **Number of Days** | **0** | **15** | **30** |
|---|---|---|---|
| **Time (hours)** | **Percentage of Guaifenesin Release** | | |
| 1 | 33 | 30 | 44 |
| 2 | 44 | 40 | 53 |
| 4 | 58 | 54 | 67 |
| 6 | 70 | 68 | 84 |
| 8 | 81 | 78 | 96 |
| 12 | 97 | 91 | 106 |

From the above *in-vitro* release data, it is evident that the extended release liquid composition prepared according to Example 1 provides the substantially similar *in-vitro* release for 30 days.

The dual chamber bottle containing suspension powder for reconstitution and vehicle of Example 1 was kept for six months at accelerated conditions i.e., 40°C/75% R.H. After six months, the powder for suspension was reconstituted with vehicle to form the extended release liquid composition and this extended release liquid composition was kept for 48 days at room temperature. The *in-vitro* dissolution was determined at 0 and 48 days using USP type II apparatus at 50 rpm, in 900 mL of 0.1N HCL at 37°C. The results of the release studies are represented in Table 2.

**Table 2: Percentage (%) of the In-Vitro Guaifenesin Release in USP Type II Apparatus (Media: 0.1N HCL, 900 mL, and 50 rpm)**

| **Number of Days After Reconstitution** | **0** | **48** |
|---|---|---|
| **Time (hours)** | **Percentage of Guaifenesin Release** | |
| 1 | 37 | 43 |
| 2 | 46 | 49 |
| 4 | 58 | 61 |
| 6 | 69 | 71 |
| 8 | 77 | 80 |
| 12 | 91 | 95 |

From the above data, it is clear that the extended release suspension powder prepared according to Example 1 stored at accelerated conditions for 6 month, upon reconstitution and storage for 48 days at room temperature, provides substantially similar *in*-*vitro*guaifenesin release for 48 days.

### Assay Data

The dual chamber bottle containingextended release powder for reconstitution and vehicle of Example 1 was kept for six months at accelerated conditions *i.e.,* 40°C/75% R.H. After one month, three months and six months the powder for suspension was reconstituted with vehicle to form the extended release liquid compositions. The assay of extended release liquid compositions of guaifenesin was determined at day 0 using a validated HPLC method.

**Table 3:Assay of Guaifenesin Extended Release Suspension**

| **Condition** | **% Assay (After reconstitution)** |
|---|---|
| Initial | 101.8 |
| 1 month (40°C/75% R.H.) | 98.4 |
| 3 months (40°C/75% R.H.) | 103.1 |
| 6 months (40°C/75% R.H.) | 99.8 |

It is evident from the above data that the extended release liquid composition prepared as per Example 1 is stable.

### Example 2: Extended Release Suspension Composition of Guaifenesin

| **Ingredients** | **Quantity (mg/mL)** |
|---|---|
| **Drug layered cores** | |
| Guaifenesin | 70.00 |
| Microcrystalline cellulose | 56.00 |
| Sodium alginate | 7.00 |
| Purified water* | q.s. |
| Weight of drug layeredcores | 133.00 |

| **Extended release coating** | |
|---|---|
| Drug layered cores | 133.00 |
| Ethyl cellulose | 39.10 |
| Dibutyl sebacate | 0.80 |
| Acetone* | q.s. |
| Purified water* | q.s. |
| Weight of extended release coated cores | 172.90 |

| **Lubrication** | |
|---|---|
| Extended release coated cores | 172.90 |
| Magnesium stearate | 0.86 |
| Weight of lubricated extended release coated cores | 173.76 |

| **Vehicle** | |
|---|---|
| Guaifenesin (Immediate release portion) | 30.00 |
| Microcrystalline cellulose/carboxymethyl cellulose sodium (Avicel CL-611) | 20.00 |
| Xanthan gum | 1.50 |
| Colloidal silicon dioxide | 3.50 |
| Sucralose | 0.50 |
| Xylitol | 450.00 |
| Strawberry flavor | 1.00 |
| Purified water | q.s. to 1 mL |

| | |
|---|---|
| *Evaporates during processing | |

### Procedure:

1. Sodium alginate was dissolved in water to a clear solution
2. Guaifenesin was dissolved in the solution of step 1.
3. Microcrystalline cellulose spheres were coated with the solution of step 2.
4. Ethyl cellulose and dibutyl sebacate were dispersed in a mixture of acetone and purified water.
5. The drug layered cores of step 3 were coated with the coating dispersion of step 4 to obtain the extended release coated cores.
6. Magnesium stearate was mixed with the extended release coated cores of step 5 to form the suspension powder for reconstitution.
7. Xylitol, guaifenesin (immediate release portion), xanthan gum, microcrystalline cellulose and carboxymethyl cellulose sodium(Avicel CL -611), colloidal silicon dioxide and sucralose were dispersed/dissolved in purified water to form a vehicle.
8. The extended release coated coresof step 6 and vehicle of step 7 were mixed to form the extended release suspension of guaifenesin.

The ready-to-use extended release liquid composition of guaifenesin can be packed in a bottle.

### Example 3: Extended Release Suspension Powder of Guaifenesin for Reconstitution

| **Ingredients** | **Quantity (mg/mL)** |
|---|---|
| **Drug layered cores** | |
| Guaifenesin | 70.00 |
| Microcrystalline cellulose | 56.00 |
| Sodium alginate | 7.00 |
| Purified water* | q.s. |
| Weight of drug layered cores | 133.00 |

| **Extended release coating** | |
|---|---|
| Drug layered cores | 133.00 |
| Ethyl cellulose | 39.10 |
| Dibutyl sebacate | 0.80 |
| Acetone* | q.s. |
| Purified water* | q.s. |
| Weight of extended release coated cores | 172.90 |

| **Lubrication** | |
|---|---|
| Extended release coated cores | 172.90 |
| Magnesium stearate | 0.86 |
| Weight of lubricated extended release coated cores | 173.76 |

| **Vehicle** | |
|---|---|
| Guaifenesin (Immediate release portion) | 30.00 |
| Microcrystalline cellulose/carboxymethyl cellulose sodium (Avicel® CL-611) | 20.00 |
| Xanthan gum | 1.50 |
| Colloidal silicon dioxide | 3.50 |
| Sucralose | 0.50 |
| Xylitol | 450.00 |
| Strawberry flavor | 1.00 |
| Purified water | q.s. to 1 mL |

| | |
|---|---|
| *Evaporates during processing | |

### Procedure:

1. Sodium alginate was dissolved in water to a clear solution
2. Guaifenesin was dissolved in the solution of step 1.
3. Microcrystalline cellulose spheres were coated with the solution of step 2 to obtain drug layered cores.
4. Ethyl cellulose and dibutyl sebacate were dispersed in a mixture of acetone and purified water.
5. The drug layered cores of step 3 were coated with the coating dispersion of step 4 to obtain the extended release coated cores.
6. Magnesium stearate was mixed with the extended release coated cores of step 5.
7. The coated cores of step 6, xylitol, guaifenesin (immediate release portion), xanthan gum, microcrystalline cellulose and carboxymethyl cellulose sodium (Avicel® CL -611), colloidal silicon dioxide, and sucralose were mixed to form suspension powder for reconstitution.
8. The suspension powder for reconstitution of step 7 was packed in a bottle.

Before administration, the suspension powder for reconstitution is mixed with purified water to form the extended release liquid composition of guaifenesin.

### Example 4: Extended Release Suspension Powder of Guaifenesin for Reconstitution

| **Ingredients** | **Quantity (In mg/mL)** |
|---|---|
| Microcrystalline Cellulose | 40.893 |

| **A. Drug Layered Cores-1** | |
|---|---|
| Guaifenesin | 75.000 |
| Sodium Alginate | 7.500 |
| Mannitol | 15.00 |
| Purified water* | q.s |

| **B. Extended Release Coating** | |
|---|---|
| Ethyl cellulose | 58.509 |
| Dibutyl Sebacate | 1.194 |
| Acetone* | q.s |
| Purified water * | q.s. |

| **C. Drug Layered Cores-2** | |
|---|---|
| Guaifenesin | 25.000 |
| Sodium Alginate | 2.500 |
| Purified water* | q.s |

| **C. Lubrication** | |
|---|---|
| Magnesium stearate | 1.153 |
| Total weight of lubricated modified release cores | 226.749 |

| **Vehicle** | |
|---|---|
| Microcrystalline cellulose and carboxymethyl cellulose sodium (Avicel CL-611) | 20.000 |
| Xanthan Gum | 1.000 |
| Colloidal Silicon dioxide | 3.500 |
| Sucralose | 0.500 |
| Xylitol | 550.000 |
| Sodium benzoate | 2.000 |
| Citric acid monohydrate | 1.000 |
| Raspberry Flavor Composite | 1.500 |
| Purified water | 403.40 |
| **Total dispersion** | **1210.15** |

### Procedure:

1. Sodium alginate was dissolved in water to a clear solution
2. Guaifenesin was added in the solution of step 1 to get dispersion.
3. Mannitol was added in above dispersion of step 2 under stirring and the dispersion was heated to 35-45 degree Celsius to get a clear solution
4. Microcrystalline cellulose spheres were coated with the solution of step 3 to obtain drug layered cores.
5. Ethyl cellulose and dibutyl sebacate were dispersed in a mixture of acetone and purified water.
6. The drug layered cores of step 4 were coated with the coating dispersion of step 5 to obtain the extended release coated cores.
7. Sodium alginate was again dissolved in water to a clear solution
8. Guaifenesin was added in the solution of step 7 to get dispersion.
9. Mannitol was added in above dispersion of step 8 under stirring and the dispersion was heated to 35-45° C to get a clear solution
10. The extended release drug layered cores of step 6 were coated with the coating dispersion of step 9 to obtain the immediate release coating over extended release coated cores.
11. Magnesium stearate was mixed with the extended release coated cores of step 10.
12. Xylitol, xanthan gum, microcrystalline cellulose and carboxymethyl cellulose sodium(Avicel® CL -611), colloidal silicon dioxide, and sucralose were dispersed/dissolved in purified water to form a vehicle.
13. The suspension powder for reconstitution of step 11 and vehicle of step 12 were filled separately in a dual chamber bottle.

### Example 5: Extended Release Suspension Powder of Guaifenesin for Reconstitution

| **Ingredients** | **Quantity (Inmg/mL)** |
|---|---|
| Microcrystalline Cellulose | 40.893 |

| **A. Drug Layered Cores-1** | |
|---|---|
| Guaifenesin | 75.000 |
| Sodium Alginate | 7.500 |
| Mannitol | 15.00 |
| Purified water * | q.s |

| **B. Extended Release Coating** | |
|---|---|
| Ethyl cellulose | 58.509 |
| Dibutyl Sebacate | 1.194 |
| Acetone* | q.s |
| Purified water* | q.s |

| **C. Drug Layered Cores-2** | |
|---|---|
| Guaifenesin | 25.000 |
| Sodium Alginate | 2.500 |
| Purified water* | Q.S. |

| **C. Lubrication** | |
|---|---|
| Magnesium stearate | 1.153 |
| Total weight of lubricated modified release cores | 226.749 |

| **Vehicle** | |
|---|---|
| Microcrystalline cellulose and carboxymethyl cellulose sodium (Avicel CL-611) | 20.000 |
| Xanthan Gum | 1.000 |
| Colloidal Silicon dioxide | 3.500 |
| Sucralose | 0.500 |
| Xylitol | 550.000 |
| Sodium benzoate | 2.000 |
| Citric acid monohydrate | 1.000 |
| Raspberry Flavor Composite | 1.500 |
| Purified water | 403.40 |
| **Total dispersion** | **1210.15** |

### Procedure:

1. Sodium alginate was dissolved in water to a clear solution
2. Guaifenesin was added in the solution of step 1 to get dispersion.
3. Mannitol was added in above dispersion of step 2 under stirring and the dispersion was heated to 35-45 degree Celsius to get a clear solution
4. Microcrystalline cellulose spheres were coated with the solution of step 3 to obtain drug layered cores.
5. Ethyl cellulose and dibutyl sebacate were dispersed in a mixture of acetone and purified water.
6. The drug layered cores of step 4 were coated with the coating dispersion of step 5 to obtain the extended release coated cores.
7. Sodium alginate was again dissolved in water to a clear solution and Guaifenesin was added in this solution to get dispersion.
8. Mannitol was added in above dispersion of step 7 under stirring and the dispersion was heated to 35-45° C to get a clear solution
9. The extended release drug layered cores of step 6 were coated with the coating dispersion of step 8 to obtain the immediate release coating over extended release coated cores.
10. Magnesium stearate was mixed with the extended release coated cores of step 9.
11. The coated cores of step 11, xylitol, xanthan gum, microcrystalline cellulose and carboxymethyl cellulose sodium(Avicel® CL -611), colloidal silicon dioxide, and sucralose were mixed to form the suspension powder for reconstitution.
12. The suspension powder for reconstitution of step 11 was packed in a bottle.

Before administration, the suspension powder for reconstitution is mixed with purified water to form the extended release liquid composition of guaifenesin.

### Examples 6-7: Extended Release Suspension Powder of Guaifenesin for Reconstitution

| **Ingredients** | **Quantity (mg/mL)** | |
|---|---|---|
| | **Example 6** | **Example 7** |
| **Drug layered cores** | | |
| Guaifenesin | 84.00 | 84.00 |
| Microcrystalline cellulose | 67.20 | 67.20 |
| Sodium alginate | 8.40 | 8.40 |
| Mannitol | 16.80 | 16.80 |
| Purified water* | q.s. | q.s. |

| **Extended release coating** | | |
|---|---|---|
| Ethyl cellulose | 104.12 | 89.70 |
| Dibutyl sebacate | 2.13 | 1.83 |
| Acetone* | q.s. | q.s. |
| Purified water* | q.s. | q.s. |

| **Lubrication** | | |
|---|---|---|
| Magnesium stearate | 1.41 | 1.41 |

| **Vehicle** | | |
|---|---|---|
| Guaifenesin (Immediate release portion) | 16.00 | 16.00 |
| Microcrystalline cellulose/carboxymethyl cellulose sodium (Avicel® CL-611) | 20.00 | 20.00 |
| Xanthan gum | 1.50 | 1.50 |
| Colloidal silicon dioxide | 3.50 | 3.50 |
| Sucralose | 0.50 | 0.50 |
| Xylitol | 550.00 | 550.00 |
| Purified water | q.s. to 1 mL | q.s. to 1 mL |

| | | |
|---|---|---|
| *Evaporates during processing | | |

### Procedure:

1. Sodium alginate, mannitol, and guaifenesin were dissolved in water to a clear solution
2. Microcrystalline cellulose spheres were coated with the solution of step 1 to obtain drug layered cores.
3. Ethyl cellulose and dibutyl sebacate were dispersed in a mixture of acetone and purified water.
4. The drug layered cores of step 2 were coated with the coating dispersion of step 3 to obtain the extended release coated cores.
5. Magnesium stearate was mixed with the extended release coated cores of step 4 to form the suspension powder for reconstitution.
6. Xylitol, guaifenesin (immediate release portion), xanthan gum, microcrystalline cellulose and carboxymethyl cellulose sodium(Avicel® CL -611), colloidal silicon dioxide, and sucralose were dispersed/dissolved in purified water to form a vehicle.
7. The suspension powder for reconstitution of step 5 and vehicle of step 6 were filled separately in a dual chamber pack.

Before administration, the contents of the dual chamber pack can be mixed to form the low dose extended release liquid composition of guaifenesin.

### Examples 8-13: Low doseextended release suspension compositions of guaifenesin.

Compositions of Examples 8-13 were prepared with low doses of guaifenesin equivalent to 700, 900, 1000, 1080 and 1100 mg of guaifenesin.

| **Ingredients** | **Quantity (mg/mL)** | | | | | |
|---|---|---|---|---|---|---|
| **Example** | **8** | **9** | **10** | **11** | **12** | **13** |
| **Drug layered cores** | | | | | | |
| Guaifenesin | 60.48 | 75.60 | 90.72 | 84.00 | 84.00 | 84.00 |
| Microcrystalline cellulose | 48.38 | 60.48 | 72.576 | 67.20 | 67.20 | 67.20 |
| Sodium alginate | 6.05 | 7.56 | 9.072 | 8.40 | 8.40 | 8.40 |
| Mannitol | 12.10 | 15.12 | 18.144 | 16.80 | 16.80 | 16.80 |
| Purified water* | q.s. | q.s. | q.s. | q.s. | q.s. | q.s. |

| **Extended release coating** | | | | | | |
|---|---|---|---|---|---|---|
| Ethyl cellulose | 74.97 | 93.71 | 112.45 | 104.12 | 104.12 | 104.12 |
| Dibutyl sebacate | 1.53 | 1.92 | 2.3004 | 2.13 | 2.13 | 2.13 |
| Acetone* | q.s. | q.s. | q.s. | q.s. | q.s. | q.s. |
| Purified water* | q.s. | q.s. | q.s. | q.s. | q.s. | q.s. |
| Magnesium stearate | 1.02 | 1.27 | 1.5228 | 1.41 | 1.41 | 1.41 |

| **Vehicle** | | | | | | |
|---|---|---|---|---|---|---|
| Guaifenesin (Immediate release portion) | 11.52 | 14.40 | 17.28 | 16.00 | 16.00 | 16.00 |
| Xylitol | 550.00 | 550.00 | 550.00 | 550.00 | 550.00 | 550.00 |
| Microcrystalline cellulose/carboxymethyl cellulose sodium (Avicel® CL-611) | 20.00 | 20.00 | 20.00 | 20.00 | 20.00 | 20.00 |
| Xanthan gum | 1.50 | 1.50 | 1.50 | 1.50 | 1.50 | 1.50 |
| Sucralose | 0.50 | 0.50 | 0.50 | 0.50 | 0.50 | 0.50 |
| Colloidal silicon dioxide | 3.50 | 3.50 | 3.50 | 3.50 | 3.50 | 3.50 |
| Purified water | q.s. to 1 mL | q.s. to 1 mL | q.s. to 1 mL | q.s. to 1 mL | q.s. to 1 mL | q.s. to 1 mL |
| **% dose reduction (based on 1200 mg dose)** | 40.00 | 25.00 | 10.00 | 41.67 | 16.67 | 8.33 |

| | | | | | | |
|---|---|---|---|---|---|---|
| *Evaporates during processing | | | | | | |

### Procedure:

1. Sodium alginate, mannitol, and guaifenesin were dissolved in water to a clear solution
2. Microcrystalline cellulose spheres were coated with the solution of step 1 to obtain drug layered cores.
3. Ethyl cellulose and dibutyl sebacate were dispersed in a mixture of acetone and purified water.
4. The drug layered cores of step 2 were coated with the coating dispersion of step 3 to obtain the extended release coated cores.
5. Magnesium stearate was mixed with the extended release coated cores of step 4 to form the suspension powder for reconstitution.
6. Xylitol, guaifenesin (immediate release portion), xanthan gum, microcrystalline cellulose and carboxymethyl cellulose sodium(Avicel® CL -611), colloidal silicon dioxide, and sucralose were dispersed/dissolved in purified water to form a vehicle.
7. The suspension powder for reconstitution of step 5 and vehicle of step 6 were filled separately in a dual chamber pack.

Before administration, the contents of the dual chamber pack can be mixed to form the low dose extended release liquid composition of guaifenesin.

### Example 14: Extended Release Suspension Powder of Guaifenesin for Reconstitution

| **Ingredients** | **Quantity (mg/mL)** |
|---|---|
| **Drug layered cores** | |
| Guaifenesin | 70.00 |
| Microcrystalline cellulose | 55.00 |
| Guar gum | 8.00 |
| Purified water* | q.s. |
| Weight of drug layered cores | 133.00 |

| **Extended release coating** | |
|---|---|
| Drug layered cores | 133.00 |
| Ethyl cellulose | 39.10 |
| Dibutyl sebacate | 0.80 |
| Acetone* | q.s. |
| Purified water* | q.s. |
| Weight of extended release coated cores | 172.90 |

| **Lubrication** | |
|---|---|
| Extended release coated cores | 172.90 |
| Magnesium stearate | 0.86 |
| Weight of lubricated extended release coated cores | 173.76 |

| **Vehicle** | |
|---|---|
| Guaifenesin (Immediate release portion) | 30.00 |
| Microcrystalline cellulose/carboxymethyl cellulose sodium (Avicel® CL-611) | 20.00 |
| Xanthan gum | 1.50 |
| Colloidal silicon dioxide | 3.50 |
| Sucralose | 0.50 |
| Xylitol | 450.00 |
| Strawberry flavor | 1.00 |
| Purified water | q.s. to 1 mL |

| | |
|---|---|
| *Evaporate during processing | |

### Procedure:

1. Guar gum is dissolved in water to a clear solution
2. Guaifenesin is dissolved in the solution of step 1.
3. Microcrystalline cellulose spheres are coated with the solution of step 2 to obtain drug layered cores.
4. Ethyl cellulose and dibutyl sebacate are dispersed in a mixture of acetone and purified water.
5. The drug layered cores of step 3 are coated with the coating dispersion of step 4 to obtain the extended release coated cores.
6. Magnesium stearate is mixed with the extended release coated cores of step 5.
7. The coated cores of step 6, xylitol, guaifenesin (immediate release portion), xanthan gum, microcrystalline cellulose and carboxymethyl cellulose sodium(Avicel® CL -611), colloidal silicon dioxide, strawberry flavor, and sucralose are mixed to form the suspension powder for reconstitution.
8. The suspension powder for reconstitution of step 7 is packed in a bottle.

Before administration, the suspension powder for reconstitution is mixed with purified water to form the extended release liquid composition of guaifenesin.

### Example 15: Extended Release Suspension Powder of Guaifenesin for Reconstitution for Pediatrics

| **Ingredients** | **Quantity (mg/mL)** |
|---|---|
| **Drug layered cores** | |
| Guaifenesin | 18.75 |
| Microcrystalline cellulose | 10.22 |
| Sodium alginate | 1.88 |
| Mannitol | 3.75 |
| Purified water* | q.s. |
| Weight of drug layered cores | 34.60 |

| **Extended release coating** | |
|---|---|
| Ethyl cellulose | 14.58 |
| Dibutyl sebacate | 0.30 |
| Acetone* | q.s. |
| Purified water* | q.s. |
| Weight of extended release coated cores | 49.48 |

| **Lubrication** | |
|---|---|
| Extended release coated cores | 49.48 |
| Magnesium stearate | 0.25 |
| Weight of lubricated extended release coated cores | 49.73 |

| **Vehicle** | |
|---|---|
| Guaifenesin (Immediate release portion) | 6.25 |
| Microcrystalline cellulose/carboxymethyl cellulose sodium (Avicel® CL-611) | 20.00 |
| Xanthan gum | 1.50 |
| Colloidal silicon dioxide | 3.50 |
| Sucralose | 0.50 |
| Xylitol | 550.00 |
| Strawberry flavor | 1.00 |
| Purified water | q.s. to 1 mL |

| | |
|---|---|
| *Evaporates during processing | |

### Procedure:

1. Sodium alginate is dissolved in water to a clear solution
2. Guaifenesin is dissolved in the solution of step 1.
3. Microcrystalline cellulose spheres are coated with the solution of step 2 to obtain drug layered cores.
4. Ethyl cellulose and dibutyl sebacate are dispersed in a mixture of acetone and purified water.
5. The drug layered cores of step 3 are coated with the coating dispersion of step 4 to obtain the extended release coated cores.
6. Magnesium stearate is mixed with the extended release coated cores of step 5.
7. The coated cores of step 6, xylitol, guaifenesin (immediate release portion), xanthan gum, Avicel® CL -611, colloidal silicon dioxide, strawberry flavor, and sucralose are mixed to form the suspension powder for reconstitution.
8. The suspension powder for reconstitution of step 7 is packed in a bottle.

Before administration, the suspension powder for reconstitution is mixed with purified water to form the extended release liquid composition of guaifenesin.

### Example 16

| **Ingredients** | **Quantity (mg/mL)** |
|---|---|
| **Drug layered cores** | |
| Guaifenesin | 75.00 |
| Microcrystalline cellulose | 40.89 |
| Sodium alginate | 7.50 |
| Mannitol | 15.00 |
| Purified water* | q.s. |

| **Extended release coating** | |
|---|---|
| Ethyl cellulose | 58.51 |
| Dibutyl sebacate | 1.19 |
| Acetone* | q.s. |
| Purified water* | q.s. |

| **Lubrication** | |
|---|---|
| Magnesium stearate | 1.00 |

| **Vehicle** | |
|---|---|
| Guaifenesin (Immediate release portion) | 25.00 |
| Microcrystalline cellulose/carboxymethyl cellulose sodium (Avicel CL-611) | 20.00 |
| Xanthan gum | 1.50 |
| Colloidal silicon dioxide | 3.50 |
| Sucralose | 0.50 |
| Xylitol | 550.00 |
| Purified water | q.s. to 1 mL |

| | |
|---|---|
| *Evaporates during processing | |

### Procedure:

1. Sodium alginate, mannitol, and guaifenesin were dissolved in water to form a solution.
2. Microcrystalline cellulose spheres were coated with the solution of step 1 to obtain drug layered cores.
3. Ethyl cellulose and dibutyl sebacate were dispersed in a mixture of acetone and purified water.
4. The drug layered cores of step 2 were coated with the coating dispersion of step 3 to obtain the extended release coated cores.
5. Magnesium stearate was mixed with the extended release coated cores of step 4 to form the suspension powder for reconstitution.
6. Xylitol, guaifenesin (immediate release portion), xanthan gum, microcrystalline cellulose and carboxymethyl cellulose sodium(Avicel® CL -611), colloidal silicon dioxide, and sucralose were dispersed/dissolved in purified water to form a Vehicle
7. The suspension powder for reconstitution of step 5 and Vehicle of step 6 were filled separately in a dual chamber pack.

### Example 17:

| **Ingredients** | **Quantity (in mg/mL)** |
|---|---|
| Microcrystalline Cellulose | 41.25 |

| **A. Drug layered Cores** | |
|---|---|
| Guaifenesin | 75.00 |
| Sodium Alginate | 7.500 |
| Mannitol | 15.00 |
| Purified water | q.s.. |

| **B. Extended Release Coating** | |
|---|---|
| Ethyl cellulose | 58.47 |
| Dibutyl Sebacate | 1.19 |
| Acetone* | q.s. |
| Purified water * | q.s. |

| **C. Lubrication** | |
|---|---|
| Magnesium stearate | 1.98 |

| **Weight of lubricated modified release coated cores** | 200.39 |
|---|---|
| **Vehicle** | |
| Guaifenesin (Immediate Release Portion) | 25.00 |
| Microcrystalline cellulose and carboxymethyl cellulose sodium (Avicel® CL -611) | 15.00 |
| Xanthan Gum | 1.50 |
| Colloidal Silicon dioxide | 3.50 |
| Sucralose | 3.00 |
| Xylitol | 415.00 |
| Glycerin | 125.00 |
| Sodium chloride | 4.00 |
| Titanium dioxide | 3.00 |
| Sodium benzoate | 2.00 |
| Citric acid monohydrate | 6.56 |
| Raspberry Flavor | 1.00 |
| Strawberry Flavor | 1.00 |
| Special compound 501437T | 2.75 |
| Purified water* | 405.08 |
| **Total** | **1213.79** |

| | |
|---|---|
| *Evaporates during processing | |

### Procedure:

1. Sodium alginate, mannitol, and guaifenesin were dissolved in water to form a solution.
2. Microcrystalline cellulose spheres were coated with the solution of step 1 to obtain drug layered cores.
3. Ethyl cellulose and dibutyl sebacate were dispersed in a mixture of acetone and purified water.
4. The drug layered cores of step 2 were coated with the coating dispersion of step 3 to obtain the extended release coated cores.
5. Magnesium stearate was mixed with the extended release coated cores of step 4 to form the suspension powder for reconstitution.
6. Xylitol, guaifenesin (immediate release portion), xanthan gum, Microcrystalline cellulose and carboxymethyl cellulose sodium(Avicel® CL -611), colloidal silicon dioxide, sucralose, Sodium benzoate and citric acid mono hydrate, glycerin, sodium chloride and titanium dioxide and raspberry flavor, strawberry flavor, special compound 501437A and were dispersed/dissolved in purified water to form a Vehicle.
7. The suspension powder for reconstitution of step 5 and Vehicle of step 6 were filled separately in a dual chamber pack.

### Multimedia dissolution studiesof the extended release liquid composition of guaifenesin prepared as per Example 16

For multimedia *in-vitro* dissolution studies, the contents of the dual chamber pack of the composition prepared according to Example 16 were mixed to form the extended release liquid composition of guaifenesin.

The multimedia *in-vitro* dissolution of the extended release liquid composition of guaifenesin prepared as per Example 16 was determined using USP type II apparatus at 100 rpm, in 900 mL of 0.1N HC/ acetate buffer (pH 4.5)/ phosphate buffer (pH 6.8) at 37°C. The results are represented in Table 4.

**Table 4: Percentage (%) of the In-Vitro Guaifenesin Release in USP Type II Apparatus (Media: 0.1N HCL/acetate buffer (pH 4.5) /phosphate buffer (pH 6.8), 900 mL, and 100 rpm)**

| **Time (hour)** | **Media** | | |
|---|---|---|---|
| | **0.1 N HCl** | **Acetate buffer (pH 4.5)** | **Phosphate buffer (pH 6.8)** |
| 0.5 | 23 | 25 | 25 |
| 1 | 26 | 26 | 27 |
| 2 | 33 | 33 | 36 |
| 4 | 63 | 61 | 76 |
| 6 | 85 | 80 | 93 |
| 8 | 95 | 90 | 97 |
| 10 | 98 | 95 | 98 |
| 12 | 100 | 97 | 99 |

### Pharmacokinetic data

The extended release liquid composition of guaifenesin of Example 16 having a concentration of about 100 mg/mL was dosed at an amount equivalent to 1200 mg guaifenesin and the single-dose pharmacokinetics was determined in fasting and fed conditions. Eleven healthy adult subjects were enrolled in this study. The results of the study are summarized in Table5 and Table 6.

**Table5: Fasting Relative Bioavailability**

| | | **Cₘₐₓ** | **AUC₀₋ₜ** | **AUC_{0-∞}** |
|---|---|---|---|---|
| **Fasting Relative bioavailability (A/R)** | **Ratio %(A/R)** | 108.43 | 107.33 | 102.32 |
| | **90% Confidence Interval** | (87.82-133.88) | (99.71-115.53) | (94.72-110.53) |
| | **90% Confidence Interval** | (58.87-89.75) | (101.45-117.54) | (100.52-116.80) |

**Table 6: Food Effect (B/A)**

| | **AUC₀₋ₜ** | **AUC_{0-∞}** |
|---|---|---|
| **Ratio % (B/A)** | 109.20 | 108.36 |
| **90% Confidence Interval** | (101.45-117.54) | (100.52-116.80) |

Wherein **A:** Test product under Fasting Condition
**B:** Test product under Fed Condition
**R:** Mucinex® (guaifenesin) Extended-Release bi-layer Tablets 1200 mg

The test formulation of guaifenesin extended release liquid composition 100 mg/ml prepared according to Example 16 has showed comparable relative bioavailability when compared with reference product Mucinex® (1200 mg guaifenesin extended-release bi-layer tablets) of Reckitt Benckiser, USA, in healthy adult human subjects under fasting condition. Test formulation when administered with high-fat and high-calorie and the results showed that there is reduced food effect on the extended release liquid composition of guaifenesin as there is comparable AUC under fasting and fed conditions.

### Dissolution studies of the extended release liquid composition of guaifenesin prepared as per Example 17 at accelerated stability storage conditions 40±2°C & 75± 5 %RH

The dual chamber bottle containing extended release powder for reconstitution and vehicle of Example 17 was kept for 2 months (60 days) at accelerated conditions *i.e.,* 40°C/75% R.H. After one month and two months, the powder for suspension was reconstituted with vehicle to form the extended release liquid compositions. The dissolution profile of extended release liquid compositions of guaifenesin was determined for initial, 1 month and 3 monthsafter reconstitution.

**Table 7: Percentage (%) of the In-Vitro Guaifenesin Release in USP Type II Apparatus (Media: 0.1N HCL, 900 mL, and 100 rpm)**

| **Time (hour)** | **0 Day** | | **30 Day** | | | **60 Day** | | |
|---|---|---|---|---|---|---|---|---|
| | **Initial** | **3 M** | **Initial** | **1M** | **3 M** | **Initial** | **1M** | **3 M** |
| 0.5 | 27 | 28 | 28 | 27 | 28 | 25 | 33 | 28 |
| 1 | 30 | 31 | 31 | 31 | 30 | 29 | 36 | 31 |
| 2 | 37 | 39 | 38 | 38 | 38 | 40 | 38 | 38 |
| 4 | 58 | 61 | 61 | 58 | 57 | 72 | 58 | 60 |
| 6 | 77 | 79 | 80 | 76 | 75 | 89 | 76 | 78 |
| 8 | 90 | 90 | 93 | 89 | 86 | 96 | 88 | 89 |
| 10 | 97 | 95 | 99 | 96 | 94 | 100 | 95 | 95 |
| 12 | 101 | 98 | 103 | 99 | 98 | 101 | 98 | 98 |

### Assay Data

The dual chamber bottle containing extended release powder for reconstitution and vehicle of Example 17 was kept for 3 months at accelerated conditions *i.e.,* 40°C/75% R.H. After one month and three months, the powder for suspension was reconstituted with vehicle to form the extended release liquid compositions. The assay of extended release liquid compositions of guaifenesin was determined at day 0, day 30 and day 60 after reconstitution using a validated HPLC method.

**Table 8: Assay of Guaifenesin Extended Release Suspension**

| **Condition** | **% Assay (After reconstitution)** | | |
|---|---|---|---|
| | 0 day | Day 30 | Day 60 |
| Initial | 100.7 | 103.0 | 103.9 |
| 1 month (40°C/75% R.H.) | 101.0 | 100.2 | 99.2 |
| 3 months (40°C/75% R.H.) | 100.9 | 99.6 | 100.6 |

It is evident from the above data that the extended release liquid composition prepared as per Example 17 is stable at accelerated conditions.

### Stability Studies

The dual chamber bottle containing extended release powder for reconstitution and vehicle of Example 17 was kept for 3 months at accelerated conditions *i.e.,* 40°C/75% R.H. After one month and three months, the powder for suspension was reconstituted with vehicle to form the extended release liquid compositions. The levels of Total related substances (RS) was determined at day 0, day 30 and day 60 after reconstitution. These are represented in Table 9 below.

**Table 9: Stability results of Guaifenesin Extended Release Suspension prepared as per Example 17**

| | **Total RS** | | |
|---|---|---|---|
| **Specification** | **0 day** | **30 day** | **60 day** |
| **Initial** | 100.7 | 103.0 | 103.9 |
| **1 Month** | 101.0 | 100.2 | 99.2 |
| **3 Month** | 100.9 | 99.6 | 100.6 |

As seen from the above Table 9, equivalent stability results have been obtained.

## Claims

1. An extended release liquid composition of guaifenesin comprising:
(i) an extended release portion comprising cores of guaifenesin having coating of one or more release controlling excipients,
(ii) an immediate release portion, and
(iii) a pharmaceutically acceptable vehicle,
wherein guaifenesin is present in an amount of 75 mg/ml to 200 mg/ml,
wherein guaifenesin is present in the cores or is layered over inert particles to form drug coated cores,
wherein the cores or the drug coated cores further comprise: an osmogent selected from xylitol, mannitol, glucose, lactose, sucrose or sodium chloride; and a stabilizer selected from alginic acid, sodium alginate, guar gum, locust bean gum, tragacanth, carrageenan, gum acacia, gum arabic, gellan gum, sterculia gum, karaya gum or xanthan gum.

2. The extended release liquid composition of claim 1, wherein the ratio of guaifenesin in an extended release portion to an immediate release portion is from 50:50 to 100:0.

3. The extended release liquid composition of claim 2, wherein the ratio of guaifenesin in an extended release portion to an immediate release portion is from 75:25 to 85:15.

4. The extended release liquid composition of claim 1, wherein the extended release coating thickness per unit surface area is from 3x10⁻³ to 7x10⁻³ micron/mm².

5. The extended release liquid composition of claim 1, wherein the inert particle is selected from a group consisting of a non-pareil seed, a microcrystalline cellulose sphere, a dibasic calcium phosphate bead, a mannitol bead, a silica bead, a tartaric acid pellet, and a wax based pellet.

6. The extended release liquid composition of claim 1, wherein the osmogent is a water soluble osmogent and is present in amount of 0.1 mg/ml to 50 mg/ml.

7. The extended release liquid composition of claim 1, wherein a stabilizer is present in an amount of 0.5% to 40% w/w based on the total weight of the drug coated cores.

8. The extended release liquid composition of claim 1, wherein the release-controlling excipient is selected from the group consisting of: a pH-dependent release controlling agent, a pH-independent release controlling agent, and combinations thereof.

9. The extended release liquid composition of claim 8, wherein the release-controlling excipients are present in an amount of 20% to 80% w/w based on the total weight of the coated cores.

10. The extended release liquid composition of claim 1, wherein the immediate release portion comprising guaifenesin is present in the form of a powder, a pellet, a bead, a spheroid, or a granule in the pharmaceutically acceptable vehicle, or in the form of immediate release coating over the extended release coated cores.

## Patentansprüche

1. Flüssige Zusammensetzung von Guaifenesin mit verlängerter Freisetzung, umfassend:
(i) einen Anteil mit verlängerter Freisetzung, umfassend Kerne von Guaifenesin mit einer Beschichtung aus einem oder mehreren freisetzungssteuernden Hilfsstoffen,
(ii) einen Anteil mit sofortiger Freisetzung und
(iii) ein pharmazeutisch akzeptables Vehikel,
wobei Guaifenesin in einer Menge von 75 mg/ml bis 200 mg/ml vorhanden ist,
wobei Guaifenesin in den Kernen vorhanden ist oder über inerte Teilchen geschichtet ist, um arzneimittelbeschichtete Kerne zu bilden,
wobei die Kerne oder die arzneimittelbeschichteten Kerne ferner umfassen: ein osmotisches Agens, ausgewählt aus Xylit, Mannit, Glucose, Lactose, Sucrose oder Natriumchlorid; und einen Stabilisator, ausgewählt aus Alginsäure, Natriumalginat, Guargummi,
Johannisbrotkernmehl, Traganth, Carrageen, Akaziengummi, Gummi arabicum, Gellangummi, Sterkuliagummi, Karayagummi oder Xanthangummi.

2. Flüssige Zusammensetzung mit verlängerter Freisetzung nach Anspruch 1, wobei das Verhältnis von Guaifenesin in einem Anteil mit verlängerter Freisetzung zu einem Anteil mit sofortiger Freisetzung 50 : 50 bis 100 : 0 beträgt.

3. Flüssige Zusammensetzung mit verlängerter Freisetzung nach Anspruch 2, wobei das Verhältnis von Guaifenesin in einem Anteil mit verlängerter Freisetzung zu einem Anteil mit sofortiger Freisetzung 75 : 25 bis 85 : 15 beträgt.

4. Flüssige Zusammensetzung mit verlängerter Freisetzung nach Anspruch 1, wobei die Dicke der Beschichtung mit verlängerter Freisetzung pro Oberflächeneinheit 3 x 10⁻³ bis 7 x 10⁻³ Mikrometer/mm² beträgt.

5. Flüssige Zusammensetzung mit verlängerter Freisetzung nach Anspruch 1, wobei das inerte Teilchen aus einer Gruppe ausgewählt ist, die aus einer Nonpareille, einer mikrokristallinen Cellulosekugel, einem Kügelchen aus zweibasigem Calciumphosphat, einem Mannitkügelchen, einem Siliciumdioxidkügelchen, einem Weinsäurepellet oder einem Pellet auf Wachsgrundlage besteht.

6. Flüssige Zusammensetzung mit verlängerter Freisetzung nach Anspruch 1, wobei das osmotische Agens ein wasserlösliches osmotisches Agens ist und in einer Menge von 0,1 mg/ml bis 50 mg/ml vorhanden ist.

7. Flüssige Zusammensetzung mit verlängerter Freisetzung nach Anspruch 1, wobei ein Stabilisator in einer Menge, bezogen auf das Gesamtgewicht der arzneimittelbeschichteten Kerne, von 0,5 Gew.-% bis 40 Gew.-% vorhanden ist.

8. Flüssige Zusammensetzung mit verlängerter Freisetzung nach Anspruch 1, wobei das freisetzungssteuernde Hilfsmittel aus der Gruppe ausgewählt ist, die besteht aus: einem pHabhängigen freisetzungssteuernden Mittel, einem pHunabhängigen freisetzungssteuernden Mittel und Kombinationen davon.

9. Flüssige Zusammensetzung mit verlängerter Freisetzung nach Anspruch 8, wobei die freisetzungssteuernden Hilfsmittel in einer Menge, bezogen auf das Gesamtgewicht der beschichteten Kerne, von 20 Gew.-% bis 80 Gew.-% vorhanden sind.

10. Flüssige Zusammensetzung mit verlängerter Freisetzung nach Anspruch 1, wobei der Anteil mit sofortiger Freisetzung, der Guaifenesin umfasst, in Form eines Pulvers, eines Pellets, eines Kügelchens, eines Sphäroids oder eines Körnchens in dem pharmazeutisch akzeptablen Vehikel oder in Form einer Beschichtung mit sofortiger Freisetzung über den beschichteten Kernen mit verlängerter Freisetzung vorliegt.

## Revendications

1. Composition liquide à libération prolongée de guaifénésine comprenant :
(i) une partie à libération prolongée comprenant des noyaux de guaifénésine ayant un enrobage d'un ou plusieurs excipients de contrôle de libération,
(ii) une partie à libération immédiate, et
(iii) un véhicule pharmaceutiquement acceptable,
dans laquelle la guaifénésine est présente en une quantité de 75 mg/ml à 200 mg/ml,
dans laquelle la guaifénésine est présente dans les noyaux ou est disposée en couches sur des particules inertes pour former des noyaux enrobés de médicament,
dans laquelle les noyaux ou les noyaux enrobés de médicament comprennent en outre : un agent osmotique choisi parmi le xylitol, le mannitol, le glucose, le lactose, le saccharose ou le chlorure de sodium ; et un stabilisant choisi parmi l'acide alginique, l'alginate de sodium, la gomme guar, la gomme de caroube, la gomme adragante, un carraghénane, la gomme d'acacia, la gomme arabique, la gomme de gellane, la gomme de sterculia, la gomme karaya ou la gomme de xanthane.

2. Composition liquide à libération prolongée selon la revendication 1, dans laquelle le rapport de la guaifénésine dans une partie à libération prolongée à une partie à libération immédiate est de 50:50 à 100:0.

3. Composition liquide à libération prolongée selon la revendication 2, dans laquelle le rapport de la guaifénésine dans une partie à libération prolongée à une partie à libération immédiate est de 75:25 à 85:15.

4. Composition liquide à libération prolongée selon la revendication 1, dans laquelle l'épaisseur d'enrobage à libération prolongée par unité de surface est de 3 x 10⁻³ à 7 x 10⁻³ microns/mm².

5. Composition liquide à libération prolongée selon la revendication 1, dans laquelle la particule inerte est choisie parmi un groupe constitué d'une graine de nonpareil, d'une sphère de cellulose microcristalline, d'une perle de phosphate de calcium dibasique, d'une perle de mannitol, d'une perle de silice, d'une pastille d'acide tartrique et d'une pastille à base de cire.

6. Composition liquide à libération prolongée selon la revendication 1, dans laquelle l'agent osmotique est un agent osmotique soluble dans l'eau et est présent en une quantité de 0,1 mg/ml à 50 mg/ml.

7. Composition liquide à libération prolongée selon la revendication 1, dans laquelle un stabilisant est présent en une quantité de 0,5 % à 40 % p/p sur la base du poids total des noyaux enrobés de médicament.

8. Composition liquide à libération prolongée selon la revendication 1, dans laquelle l'excipient de contrôle de libération est choisi dans le groupe constitué : d'un agent de contrôle de libération dépendant du pH, d'un agent de contrôle de libération indépendant du pH et de combinaisons de ceux-ci.

9. Composition liquide à libération prolongée selon la revendication 8, dans laquelle les excipients de contrôle de libération sont présents en une quantité de 20 % à 80 % p/p sur la base du poids total des noyaux enrobés.

10. Composition liquide à libération prolongée selon la revendication 1, dans laquelle la partie à libération immédiate comprenant de la guaifénésine est présente sous la forme d'une poudre, d'une pastille, d'une perle, d'un sphéroïde ou d'un granulé dans le véhicule pharmaceutiquement acceptable, ou sous la forme d'un enrobage à libération immédiate sur les noyaux enrobés à libération prolongée.
